Europäisches Patentamt

European Patent Office

Office européen des brevets

(19)

(11) Publication number: **0 145 922**
**A2**

(12) **EUROPEAN PATENT APPLICATION**

(21) Application number: **84113283.0**

(22) Date of filing: **08.04.82**

(51) Int. Cl.⁴: **A 61 M 3/00**, B 65 D 83/00

(30) Priority: **09.04.81 US 252558**
**29.01.82 US 344254**
**29.01.82 US 344255**

(43) Date of publication of application: **26.06.85**
**Bulletin 85/26**

(84) Designated Contracting States: **CH DE FR GB IT LI SE**

(60) Publication number of the earlier application in accordance with Art. 76 EPC: **0063891**

(71) Applicant: **DENTSPLY INTERNATIONAL INC., 570 W. College Avenue P.O. Box 872, York Pennsylvania 17405 (US)**

(72) Inventor: **Dougherty, Emery W., 451 Hunting Park Lane, York Pennsylvania 17402 (US)**
Inventor: **Welsh, Richard E., 301 Montgomery Street, Milford Delaware 19963 (US)**

(74) Representative: **Newens, Leonard Eric et al, F.J. CLEVELAND & CO. 40/43 Chancery Lane, London WC2A 1JQ (GB)**

(54) **Ejector holder and capsule-like cartridge therefor.**

(57) A capsule-like cartridge comprising a cylindrical body (10) of uniform diameter open at one end and provided with an annular flange (11) surrounding the open end (10₁), the other end being closed by an integral hemi-spherical end (10₂) of the same radius as said body and provided with an angularly extending discharge nipple (15) communicating with the interior of the hemi-spherical end and body. After the body is loaded with a precisely measured quantity of material, the open end (10₁) is closed by a piston (12₁) slidably movable along said body the opposite end being complementary in shape to the hemispherical end of the body whereby the entire measured amount of material in the body except the scintilla thereof lodged in the nipple is discharged. The body and piston are moulded from opaque material impervious to the passage of light and the tip of the nipple is closed by a cap (14) which may be colour-coded to indicate various facts as kind of material, weight or quantity, or otherwise.

## CARTRIDGE FOR VISCOUS MATERIAL

This invention relates to a cartridge for viscous material especially dental material.

In recent years it has become popular to package various types of material, especially medicinal or quasi-medicinal types in a sealed cartridge, insertable in a suitable type of holder and/or ejector device, for purposes of preserving purity of the medicament and the like, insuring for example a patient of accurately measured quantities, as well as minimizing effort now required in introducing bulk amounts of material into syringes and ejecting measured quantities thereof.

It has been found in the operation of certain devices to Dragan (see U.S. Patent Specifications 3581399 and 4198756) particularly relative to the curved discharge end of the cartridges that there have been occasions when the leading end of the ejecting plunger or the piston within the cartridge pushed through the wall adjacent the outer end of the cartridge. The present invention seeks to obviate this difficulty and also to provide what is believed to be a simple and improved compartment at the forward end of the barrel of the holder, as well as also providing an improved cartridge not subject to the difficulties of Dragan's cartridges.

According to the present invention there is provided a cartridge solely operable by being mounted upon an ejector-type holder characterised in that it comprises a hollow elongated uniformly cylindrical body of predetermined length and uniform diameter interiorly and exteriorly and moulded from a rigid plastics material, one end of said body being open and formed at the extremity thereof with an annular relatively short circular exterior flange of limited width and arranged to be detachably mounted within a complementary seat in an ejector-type holder, the opposite end of said body being closed by a hemi-spherical wall of substantially the same uniform thickness as said body, a discharge nipple of the same material as the body and moulded integrally therewith and extending from said closed end of said body at an angle to the axis of said body to facilitate directing a discharge from the cartridge, a piston having sidewalls closely complementary to the inner walls of said body and inserted into the open end thereof to form a combination closure and ejecting means for material when contained in said cartridge, the inner end of said piston being hemi-spherical and complementary in shape to the interior surface of the closed end of said body to effect injection of substantially the entire contents of said cartridge when said piston is fully inserted into said body of the cartridge, sealing means comprising a cup-shaped cap removably connected to the outer end of the discharge nipple on said body to close said outer end of the nipple to seal the contents of the cartridge against ingress of ambient atmosphere and/or any surrounding contaminating matter, and said cap being colour-coded to indicate desired properties of the contents of the cartridge.

The cartridge body may be moulded and also the piston from a plastics material, such material optionally being opaque in colour to permit loading the same with a light-sensitive material and storage thereof.

The invention will now be described by way of example only with reference to the figures of the accompanying drawing in which:-

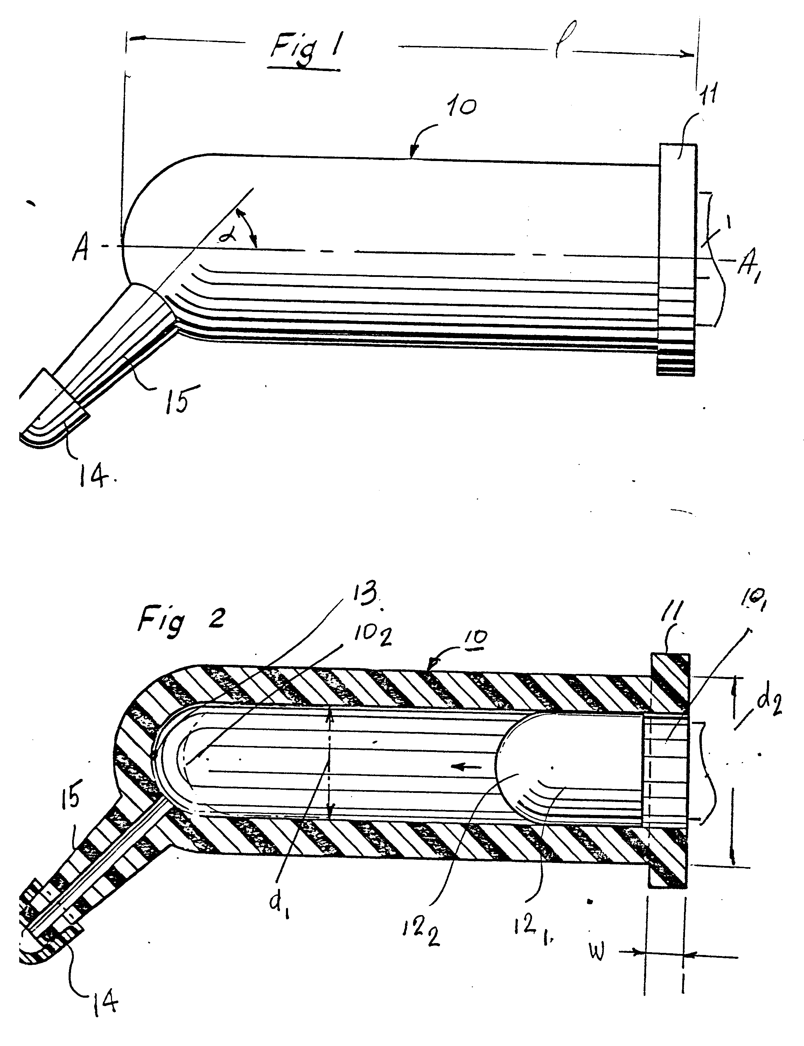

Figure 1 is a side elevation of a cartridge including a cap for the discharged nipple of the cartridge body; and

Figure 2 is a vertical section of the cartridge shown in Figure 1.

The cartridge shown generally at 10 has an annular flange 11 a piston $12_1$ with a hemi-spherical end $12_2$ that conforms to the closed end 13 of the cartridge. A cup-shaped cap 14 which is suitably shaped either frictionally to engage the tip portion of the discharge nipple 15 or either the cap or nipple, or both, may have appropriate threads formed therein or thereon to secure the cap releasably upon the tip of the nipple in sealed manner.

Moreover, the cap 14 serves an important additional feature in that, in addition to sealing the contents of the cartridge, in conjunction with the piston $12_1$, the cap also may be colour-coded for any of a number of purposes such as to indicate the kind of material for specified purposes, weight or quantity of the material therein, setting time, and otherwise.

Also, the body of the cartridge as well as the cap 14 and piston $12_1$ may all be moulded from a similar plastics material which is coloured suitably to render the items opaque or otherwise impervious to the transmission of ambient light which, if the contents are subject to being set by such light, prevents premature setting or curing thereof. The cartridge 10 is solely operable by being mounted upon an ejector-type holder (not shown). The cartridge 10 has a hollow elongated uniformly cylindrical body of predetermined length $\ell_1$ and uniform diameter interiorly $d_1$ and exteriorly $d_2$ and moulded from a rigid plastics material, one end $10_1$ of said body being open and formed at the extremity thereof with an annular relatively short circular exterior flange 11 of limited width $\underline{w}$ and arranged to be detachably mounted within a complementary seat in an ejector-type holder (not shown). The opposite end $10_2$ of said body being closed by a hemi-spherical wall 13 of substantially the same uniform thickness as said body, a discharge nipple 15 of the same material as the body and moulded integrally therewith and extending from said closed end $10_2$ of said body at an angle $\alpha$ to the axis of said body to facilitate directing a discharge from the cartridge, a piston $12_1$ having sidewalls closely complementary to the inner walls of said body and inserted into the open end thereof to form a combination closure and ejecting means for material when contained in said cartridge, the inner end of said piston $12_1$ being hemi-spherical and complementary in shape to the interior surface of the closed end of said body to effect injection of substantially the entire contents of said cartridge when said piston $12_1$ is fully inserted into said body of the cartridge, sealing means comprising a cup-shaped cap 14 removably connected to the outer end of the discharge nipple 15 on said body to close said outer end of the nipple to seal the contents of the cartridge against ingress of ambient atmosphere and/or any surrounding contaminating matter, and said cap being colour-coded to indicate desired properties of the contents of the cartridge.

In practice the nipple 15 is applied to the interior of an oral cavity of a dental patient.

CLAIMS

1. A cartridge 10 solely operable by being mounted upon an ejector-type holder characterised in that it comprises a hollow elongated uniformly cylindrical body of predetermined length $\ell$ and uniform diameter interiorly $d_1$ and exteriorly $d_2$ and moulded from a rigid plastics material, one end $10_1$ of said body being open and formed at the extremity thereof with an annular relatively short circular exterior flange 11 of limited width $\omega$ and arranged to be detachably mounted within a complementary seat in an ejector-type holder, the opposite end of said body $10_2$ being closed by a hemi-spherical wall 13 of substantially the same uniform thickness as said body, a discharge nipple 15 of the same material as the body and moulded integrally therewith and extending from said closed end of said body at an angle $\alpha$ to the axis $AA_1$ of said body to facilitate directing a discharge from the cartridge, a piston $12_1$ having sidewalls closely complementary to the inner walls of said body and inserted into the open end $10_1$ thereof to form a combination closure and ejecting means for material when contained in said cartridge, the inner end $12_2$ of said piston $12_1$ being hemispherical and complementary in shape to the interior surface of the closed end of said body to effect injection of substantially the entire contents of said cartridge when said piston $12_1$ is fully inserted into said body of the cartridge, sealing means comprising a cup-shaped cap 14 removably connected to the outer end of the discharge nipple 15 on said body to close said outer end of the nipple to seal the contents of the cartridge against ingress of ambient atmosphere and/or any surrounding contaminating matter, and said cap being colour-coded to indicate desired properties of the contents of the cartridge.

- 3 -

2.  The cartridge as claimed in Claim 1 wherein said body and piston are formed from a plastics material suitably coloured to render the same impervious to the transmission of ambient light, thereby rendering the cartridge adapted to contain light-curable material in a manner to prevent premature curing of such material while stored in said cartridge.

$1/1$

0145922

*Fig 1*

*Fig 2*